Europäisches Patentamt

European Patent Office

Office européen des brevets

(11) Publication number: **0 299 205 B1**

(12) **EUROPEAN PATENT SPECIFICATION**

(45) Date of publication of patent specification: **15.04.92**  (51) Int. Cl.⁵: **A01N 25/28**, B01J 13/02, A61K 9/52

(21) Application number: **88109276.1**

(22) Date of filing: **10.06.88**

(54) **Sustained release microcapsules and method for the production thereof.**

(30) Priority: **19.06.87 US 64820**
**19.06.87 US 64821**
**19.06.87 US 64859**
**20.07.87 US 75092**

(43) Date of publication of application:
**18.01.89 Bulletin 89/03**

(45) Publication of the grant of the patent:
**15.04.92 Bulletin 92/16**

(84) Designated Contracting States:
**BE CH DE FR GB IT LI LU NL SE**

(56) References cited:
**EP-A- 0 188 309**
**WO-A-79/00155**
**DD-C- 217 821**
**US-A- 3 959 457**
**US-A- 4 743 583**

(73) Proprietor: **TEMPLE UNIVERSITY**
**406 University Services Building**
**Philadelphia Pennsylvania 19122(US)**

(72) Inventor: **Speaker, Tycho J.**
**2112 Cherry Street**
**Philadelphia, PA 19103(US)**
Inventor: **Collett, John H.**
**17 Beccles Road Brooklands**
**Sale Cheshire Co., M333RP(GB)**
Inventor: **Chang, Frank N.**
**1412 Candlebrook Drive**
**Dresher, PA 19025(US)**
Inventor: **Harvey, William R.**
**126 Bellows Way**
**Lansdale, PA 19446(US)**
Inventor: **Speaker, Tully J.**
**2112 Cherry Street**
**Philadelphia PA 19103(US)**

(74) Representative: **Schwabe - Sandmair - Marx**
**Stuntzstrasse 16**
**W-8000 München 80(DE)**

EP 0 299 205 B1

## Description

The present invention relates to sustained release microcapsules and a method of their preparation.

Background of the Invention

It is known from U.S. Patent 3,959,457, issued May 25, 1976, that microparticles useful for the controlled release of a core substance such as a drug are obtained from an emulsion of an aqueous solution or colloidal suspension of a partially hydrophilic, partially lipophilic, polyfunctional Lewis acid and an organic, polyfunctional Lewis base solvated in a slightly polar non-aqueous solvent. As described in this patent, the disclosure of which is incorporated herein by reference, microparticles comprising the Lewis acid/Lewis base reaction product are precipitated from the emulsion as microspheroids encapsulating a core substance incorporated into the emulsion. The Lewis acids employed preferably have a molecular weight of at least about 12,000 and provide a sufficient number of spaced cross-linking sites for he polyfunctional Lewis base to provide a lattice-type encapsulating wall to permit the gradual release by diffusion of the encapsulated material through the pores thereof at the desired rate. The rate of release of a given core substance is also variable according to the wall thickness which is broadly controllable by the concentration of the reactants in the emulsion, and the reaction time. The Lewis bases employed herein have a basic strength ($pk_a$) of from about 7 to 12, and react at the interfaces of the organic and aqueous phases of the emulsion through dipole and/or ionic bonding to form the encapsulating microspheroids. For pharmaceutical use in intravenous administration, the pores of the microspheroid wall should be sufficiently large to permit the desired diffusion rate of an encapsulated pharmaceutical into the bloodstream, while substantially excluding the influx of blood plasma into the interior of the capsule to prevent denaturation of susceptible encapsulated pharmaceuticals. In such applications, it is essential to employ Lewis acids which have reactive or cross-linking sites (carboxyl groups) spaced at least about a distance equivalent to a normal carbon chain containing 20, or preferably 30, carbon atoms, and no more than a distance equivalent to a normal carbon chain containing 360, preferably 240, carbon atoms. Depending in part on wall thickness and the contemplated application, reactive sites spaced outside those ranges may be employed effectively for the desired result in specific applications, e.g., retention of encapsulated material, exclusion of ambient media, and rate of release of encapsulated material. For example, if oxidized cellulose is the selected Lewis acid reactant, the distance between carboxyl groups may be as much as 100 or as few as 4 carbon atoms in a normal carbon chain for effective sustained release of many substances.

The microparticles described in U.S. 3,959,457 are prepared by combining a solution of the selected Lewis base in a slightly polar organic solvent with an aqueous solution or colloidal suspension of the Lewis acid. To encapsulate a core substance, which must be substantially soluble in the organic phase and immiscible with the aqueous phase, this material is dissolved in the organic phase before the two phases are combined. The success of the process in forming the microparticles is dependent upon the choice of organic solvent for the Lewis base; the solvent must be sufficiently polar to anisotropically orient the salt-forming components and stabilize the product polysalt film against hydration in the aqueous phase, as described in detail in U.S. 3,959,457. In general, nucleophilic or electrophilic polar solvents useful in the preparation of the microparticles have a dielectric constant of no less than about 4 Debye units, although solvents exceeding this limit may be found useful in particular applications. It is of course necessary to select a solvent which does not appreciably deactivate a core substance.

After the reaction is completed, the phases are separated. The organic phase includes a multiplicity of shell-like structures of polysalt film, each enclosing solvent, residual unreacted polyfunctional Lewis base, and any foreign material incorporated in the organic phase, such as a pharmaceutical for which the microparticulate product is to act as a carrier. This organic phase, after separation, is conveniently washed with water which is then separated by decantation. The remaining solvent, particularly if it is a low molecular weight, low boiling solvent such as dichloromethane, is then removed as by evaporation, by drying in air, or under reduced pressure. A core material of appropriate polarity will function to maintain anisotropic orientation of the wall-forming salt films and stabilize the product as described supra. The product is a non-agglomerating microparticulate material of very small dimensions. The particle size dimensions are controlled primarily by the degree of dispersion of the emulsified reaction media or more specifically by the droplet size of the organic phase in the emulsified reaction media. Particle sizes of smaller than 2 $\mu$m are obtainable by this process. While considerable larger particles are also readily produced, for intravenous administration, particle sizes of no more than about 7 $\mu$m, approximately the size of a normal erythrocyte, are typically preferred.

Suitable exemplary Lewis acids, Lewis bases, and polar solvents for the bases useful in the practice of the present invention are as follows:

Lewis Acids

Agar, Acacia, Gum, Arabic Acid, Carboxymethylcellulose, Ghatti Gum, Guar Gum, Methylcellulose, Oxidized Cellulose, Pectin, Tragacanth and Polyethylene Gylcol.

Lewis Bases

Monofunctional amines, Hexylamine, Isopentylamine, N-Methylpiperidine, Piperidine, Difunctional amines, Dimethylethylenediamine, Hexanediamine, Piperazine, Triethylenediamine, Ethylenediamine, Polyfunctional amines, Hexamethylrosanilium cation as chloride, Rosanilium cation as chloride, Tetramethylrosanilium cation as chloride, Melamine, Tetraethylenepentamine and Triethylenetetramine.

Polar Solvents

Bromoform, Chloroform, Dichloromethane, Dichloroethane, Diethyl ether, Diisopropyl ether, Methyl ethyl ketone and Nitrobenzene.

An example of the preparation of microcapsules according to U.S. Patent 3,959,457 is as follows:

A solution of 10 g of finely divided acacia powder (USP) in 50 ml of distilled water and a second solution of 0.05 g of anhydrous piperazine and 50 ml of chloroform are prepared at room temperature. To the latter solution is added 100 mg of a selected drug, namely, Quinacrine HCl. While stirring the acacia vigorously with a magnetic stirrer, the chloroform solution is added slowly and steadily so that the chloroform solution is emulsified in the aqueous solution. Stirring is continued for about 3 minutes to produce emulsion droplets about 5 $\mu$m in diameter. The organic phase, now consisting of emulsified microspheres, is then allowed to settle to the bottom of the container and the supernatant clear, aqueous layer is decanted. The organic phase is then washed with water to remove excess chloroform, piperazine and acacia. On exposure to the atmosphere, the microspheres lose their chloroform by evaporation leaving a microparticulate material comprised of microspheroids made up of wrinkled, continuous shell-like films, surrounding the drug, Quinacrine HCl.

Microparticulate material prepared by the method of this example has been injected directly into the bloodstream of a frog and circulation of the microspheroids in the blood stream of the frog has been proven by microscopic observation of these microspheroids subsequently flowing in the capillary network of the web of the frog's foot. No adverse effect on the frog was observed. The sustained release of drug from the microparticulate material has been observed generally in dogs in vivo and has been studied in detail in vitro. The continued circulation of intravenously injected radionuclide-bearing microparticles has, in separate experiments, been demonstrated in rats.

The invention relates to a delivery system for the gradual release of a core substance from a microcapsule comprising a core and a microcapsule wall of an anisotropic salt film which is the reaction product of

(a) a Lewis base or a salt thereof having a basic strength ($pK_a$) of from about 7 to 12 and reacting at the interfaces of the organic and aqueous phases of an emulsion through dipole and/or ionic bonding, in a slightly polar organic solvent for the base or salt and an optional adjuvant, and

(b) a polyfunctional partially hydrophilic, partially lipophilic Lewis acid or salt thereof having reactive or cross-linking sites spaced about a distance equivalent to a normal carbon chain containing at least 20 and no more than 360 carbon atoms in an aqueous solution or suspension and an optional adjuvant,

characterized in that the core substance is either a pharmaceutical material or a proteinaceous material comprising a biologically-active peptide or polypeptide containing from 14 to 100 amino acids, and the slightly polar organic solvent is a non-denaturing solvent or solvent complex sufficiently polar to anisotropically orient the salt-forming components and stabilize the product salt film against hydration in the aqueous phase and capable of solubilizing or suspending the proteinaceous material without deactivation thereof.

The invention further relates to a method for the production of the above defined delivery system for the gradual release of a core substance from a microcapsule comprising a core and a microcapsule wall of an anisotropic salt film, characterized in that the delivery system is produced by reacting, in the form of an emulsion,

(a) a first reaction system comprising

(i) a first reactant mixture of a Lewis base or a salt thereof having a basic strength ($pK_a$) of from about

7 to 12 and reacting at the interfaces of the organic and aqueous phases of an emulsion through dipole and/or ionic bonding, in a slightly polar organic solvent for the base or salt, said slightly polar organic solvent being a non-denaturing solvent or solvent complex sufficiently polar to anisotropically orient the salt-forming components and stabilize the product salt film against hydration in the aqueous phase and capable of solubilizing or suspending the core substance without deactivation thereof, an optional adjuvant and a core substance, said core substance being either a pharmaceutical material or a proteinaceous material comprising a biologically-active peptide or polypeptide containing from 14 to 100 amino acids and being solubilizable or suspendable in said organic solvent, with

(ii) a second reactant mixture of a polyfunctional partially hydrophilic, partially lipophilic Lewis acid or salt thereof having reactive or cross-linking sites spaced about a distance equivalent to a normal carbon chain containing at least 20 and no more than 360 carbon atoms in an aqueous solution or suspension and an optional adjuvant; or

(b) a second reaction system comprising

(i) a first reactant mixture comprising a salt of said Lewis base in said slightly organic solvent for the base, an optional adjuvant and said core substance, with

(ii) a second reactant mixture comprising a salt of said polyfunctional Lewis acid in an aqueous solution or suspension and an optional adjuvant; or

(c) a third reaction system comprising

(i) a first reactant mixture comprising said Lewis base or a salt thereof in said slightly organic solvent for the base and said core substance, with

(ii) a second reactant mixture comprising said polyfunctional Lewis acid or a salt thereof in an aqueous solution or suspension, and with

(iii) a third reactant mixture comprising a wall-forming or core-forming adjuvant or a combination thereof;

to form a core substance microencapsulated in an anisotropic salt film through which the core substance is gradually permeable.

## Description of the Invention

According to the present invention, microparticles comprising a Lewis acid/Lewis base reaction product of the type described in U.S. Patent 3,959,457 as summarized above are employed in the encapsulation of a proteinaceous substance comprising a biologically-active peptide or polypeptide. The product provides a gradual-release protein delivery system comprising a proteinaceous material incorporated in a Lewis acid/Lewis base high molecular weight salt microparticulate material of the type referred to above, in which the polar organic manufacturing solvent is a solvent for the Lewis base wall-forming component and is also a non-denaturing solvent which is capable of effectively solubilizing a proteinaceous macromolelcule core material without deactivation thereof.

The proteinaceous materials useful herein include biologically active peptides and proteins nominally containing from 14 to 1000 amino acids, including purified or crude biological extracts thereof. Such peptides and proteins typically comprise single or multiple amino acid polymer chains which are able to diffuse out of the individual microparticulate capsules. Exemplary non-denaturing manufacturing solvents useful herein include non-cyclic esters, cyclic esters or lactones, cyclic amides and linear amides, having Hildebrand solubility parameters or Hildebrand delta values of from about 20 to about 30. Representative solvents include ethyl acetate, butyrolactone, valerolactone, caprolactone, N-methyl-gamma pyrrolidone, N-methylformamide. N-methylacetamide, N,N-dimethylformamide, N,N-dimethylacetamide, N-ethylacetamide and cyclic amides such as N-methylgamma pyrrolidone. In some instances, the non-denaturing organic solvents may be used in combination with slightly polar organic solvents of U.S. Patent 3,959,457, which have denaturing characteristics, as long as the solvent combination or complex itself does not appreciably tend to denature the encapsulated material.

Although it is widely recognized that most proteins are generally not soluble or stable in non-aqueous solvents of the type typically used in the manufacture of microparticles of the type referred to in U.S. Patent 3,949,457, organic solvents employed to produce the protein delivery system of the instant invention are capable of approximating and in some instances exceeding the ability of aqueous systems to dissolve proteins. Furthermore, these organic solvents are capable of effectively solubilizing peptides and proteins without denaturing the peptide or protein, thus preserving its biological activity. Additionally, these solvents are also suitable solvents for the Lewis base wall-forming component of the Lewis acid/Lewis base microparticle.

It is of particular note that some of the organic solvents which are useful as protein solvents for the manufacture of the microparticles of the present invention are quite water soluble/water miscible. Therefore, they differ in a fundamental characteristic from the water-immiscible organic solvents described in U.S. Patent 3,959,457. Such solvents are ordinarily not useful in the production of microcapsules. However, these water soluble/water miscible solvents may be advantageously employed as solvents in the manufacture of new microparticles containing proteins and peptides by either:

a. mixing a protein solution (e.g. growth hormone in butyrolactone) with an approximately equal volume of a water-immiscible solvent (such as dichloromethane) containing the Lewis base and emulsifying the organic solvent mixture in the wall forming step; or

b. taking advantage of the tendency of concentrated solutions of protein or peptide in certain organic solvents (e.g. growth hormone in N-methylformamide) to increase viscosity to the point of gelation and, gelled, to be much less rapidly water-miscible.

In the latter instance, a solution of Lewis base in organic solvent is used to prepare a concentrated, highly viscid solution of the peptide. This viscid, slowly water-miscible solution is then utilized in the wall-forming step as if it were truly water immiscible.

Typical of useful protein solvents, which are water-soluble/water-miscible, are butyrolactone, N-methylformamide, and N-methylacetamide. Such solvents also rapidly diffuse from the capsules once the wells have been formed. This rapid diffusion and the water solubility of these solvents also facilitate separation and removal of the organic manufacturing solvent by, for example, dialysis or centrifugation.

Whether a given solvent is suitable for solubilizing proteins depends, inter alia, on the structure of the protein peptide and the exposed solvent-interactive surface of the protein/peptide. The mechanisms by which solute molecules interact with solvent molecules to form solutions is thought to involve a process in which energy is expended. The energy expended and transferred in such a process is derived from the breaking of old and the making of new associative van der Waals, dipole, hydrogen, and ionic bonds and from the initial thermal energy of the system. If approximately equal amounts of energy are expended in weakening the old self-associations of solute and solvent molecules respectively in forming new solute-solvent associative bonds, a solute is relatively more soluble in a solvent than it is if there is a great disparity in the amounts of energy required in the various steps of the solvation/solution process. Simply stated, a solute having an energy value near that of the solvent will be more readily dissolved by that solvent than by a solvent having a much different energy value. The Hildebrand theory of solution and its intellectual descendants describe the process with mathematical rigor. Often suitable solvents for such applications may be selected from those having Hildebrand solubility parameter values (sometimes referred to as cohesive energy density or Hildebrand delta values) in the range from about 20 to about 30 MPa$^{1/2}$ - (i.e. the square root of the mega-Pascal value), as described in A.F.M. Barton's Handbook of Solubility Parameters and Other Cohesion Parameters, CRC Press, Boca Raton, FL 1983.

An exact solubility parameter range cannot be rigorously defined since examination of Barton shows that for any single substance several (usually) slightly different values of this parameter have been measured or calculated by different workers. As a guide, it may be useful to note the Hildebrand values reported for some solvents mentioned above:

| Solvent | MPa$^{1/2}$ |
| --- | --- |
| dichloromethane | 19.8 |
| N,N-diethylacetamide | 20.2 |
| N,N-diethylformamide | 21.7 |
| butyrolactone | 26.3 |
| N-ethylformamide | 28.4 |
| N-methylformamide | 32.9 |
| water | 47.9 |

Many proteins and peptides are large complex molecules capable of folding and coiling about themselves in such a manner as to expose only some parts of the molecule while masking other parts. In some instances, interaction with a solvent alters the degree or type of folding and coiling of a protein. Thus, while Hildebrand values are illustrative of the solvating capacity of a given solvent, it is the exposed solvent-interactive surface of the protein or peptide which may be a primary determinant of which organic solvent will be the most effective in dissolving the polypeptide.

Alternatively, the solute-solvent interaction may be considered in the dissolution of the polypeptide. The partial cohesive energy parameters of Hansen (which take into account other interactions such as dipole, van der Waals and hydrogen bonding contributions to cohesive energy) may also be used in selecting organic solvents with which to dissolve proteins and peptides. One preferably selects as candidate solvents those which have Hildebrand (Hansen) parameters near that of the material to be dissolved. Since these parameters are not readily (if at all) available for most proteins or peptides, they may be estimated from constituent values described by R. F. Fedors, Polym. Eng. Sci., Vol. 14, pgs. 147 and 472 (1974).

Alternatively, one may proceed by measuring the solubility of a protein in a series of organic solvents with graded Hildebrand delta values. Solute solubility will increase as solute and solvent approach one another in delta value.

The microparticle protein delivery systems of the invention are prepared according to the process of U.S. 3,959,457 as described above, by addition of a non-aqueous Lewis base in a non-denaturing solvent according to the present invention, to an aqueous solution of a Lewis acid with agitation to form an emulsion of organic phase droplets in a continuous aqueous phase. Any of the Lewis acids or bases described in U.S. 3,959,457 are suitable, with particular reference to piperazine, triethylene diamine, or ethylene diamine as bases, and acacia gum, arabic acid, and carboxymethylcellulose as acids. Monofunctional Lewis bases, such as triethylamine are also contemplated.

Included in the non-aqueous solvent in accordance with the present inventon, is a proteinaceous core material comprising proteins and polypeptides. As used herein, "polypeptide" refers to biologically-active polymeric compounds made up of from nominally 14 to 1000 amino acids which may consist of single or multiple amino acid polymer chains. These peptides and proteins are preferably soluble in the organic solvents of the present invention, although it is also possible to encapsulate partially solubilized proteins or protein suspensions using the methods and materials disclosed herein. However, use of a protein solution for encapsulation is preferable, as it allows much more careful control of the resultant particle size and ultimately the dispersability of the encapsulated compounds.

Typically, polypeptide preparations are made from a lyophilized or "freeze dried" form of a biologically-derived peptide or protein. Biologically-derived means that the sources of these peptides or proteins are usually tissue or organ preparations from which biologically active constituent material is extracted. However, it is recognized that these source of polypeptides are not limitations and that "polypeptides" as used herein is meant to include other non-conventionally derived peptides and proteins, such as may be produced by peptide synthesis or by genetically altered microorganisms.

With respect to the three-dimensional or conformational structure of these polypeptides, the protein/peptide compounds may be relatively linear or complex three dimensional structures. Representative of such compounds are growth hormone (somatotrophin), insulin and glucagon. Growth hormones of various species, for example, differ in exact amino sequence but typically have molecular weights near 30,000 atomic mass units. Insulins of various species also differ in amino acid sequence but have molecular weights in the range near 7000 atomic mass units. For purpose of release of the polypeptide core material, it must be of such molecular size and conformation that it is capable of diffusional escape from the individual microparticulate material. However, it should not be inferred that the active core material must escape rapidly. Indeed, sustained release of such bioactive material, such as growth hormone or insulin, over periods of days or weeks may be preferred. Many bioactive peptides are highly potent materials of which only small amounts are required to produce substantial effects. Thus, for example, release of only a fraction of a milligram of growth hormone per day is adequate for therapeutic purposes.

In a particular embodiment of the invention, the proteinaceous substance encapsulated in the microparticles comprises an insecticidal toxin, particularly a toxin derived from Bacillus thuringiensis. "B. thuringiensis toxin" as used herein refers to one toxin or a combination of toxins from Bacillus thuringiensis or subspecies of this organism, whether isolated in pure form or as more or less crude preparations containing other cell components, debris and spores. This embodiment of the invention especially comprises toxin delivery vehicles consisting essentially of Bacillus thuringiensis insecticidal delta endotoxins - (sometimes referred to herein as "Bt toxins").

The toxins are encapsulated as discussed above, employing a non-denaturing organic solvent in conjunction with the Lewis base.

The microcapsular delivery system for insecticidal toxins of the present invention is a dispersion of small microparticles which retain and protect enclosed toxin and which provide gradual or controlled release of toxin from the microparticles. Preferably, the microparticles have an average spherical diameter of from about 1 mm to about 1/1000 mm (1 $\mu$m). In some instances, for example, as used against mosquito larvae, the preferred microparticle size range is from 1 to 70 $\mu$m to match the food particle size preferred by these filter feeding organisms. For use against other specific types of insects, such as leaf-chewing insects, it is obvious that larger (up to 150 $\mu$m) or smaller size microparticles may be optimal.

Surprisingly, the insecticidal toxins encapsulated according to the present invention have enhanced insecticidal activity as compared to native toxin, with up to two-fold increases contemplated. This increase in potency of encapsulated toxin may be due in part to suppression of an "anti-feedant" response in insect larval populations previously exposed to native toxin, when offered insecticidal toxins microencapsulated according to the present invention.

It may be desirable to render toxin-containing microparticles of the present invention adhesive so that the microparticles adhere to the treated portions of plants on which insects feed. This may be done by treating the microparticulate material with an excess of Lewis acid such as arabic acid, gum arabic, guar gum, methyl cellulose, carboxymethylcellulose, sodium carboxymethylcellulose, hydroxypropylcellulose, cellulose sulfate or other such Lewis acids.

Insecticidal toxins in microcapsules exposed to sunlight are likely to undergo photodegradation and consequent loss of insecticidal activity. It therefore may be desirable to incorporate in the individual microcapsules, a sunscreen or sunblocking agent to prolong the insecticidal activity in the environment by protecting the toxins against photodegradation. This may be accomplished by incorporating a strongly ultra-violet absorbing substance, such as phenyl salicylate, in the microcapsules.

There are several types of toxin-promoting adjuvants which may be used in preparing insecticidal-containing microcapsules of the present invention. A first type of adjuvant is an ester or amide which aids in the encapsulation of a low-density oil or on ultra-violet absorbing sunscreen. A second type of adjuvant is a low-density oil which makes the intact microcapsules buoyant. A third type of adjuvant is an ultra-violet absorbing sunscreen which protects the insecticial toxins in intact buoyant capsules. Another type of adjuvant is an addition to increase the mechanical strength of the microcapsule. Yet another adjuvant is one which provides an adhesive characteristic to the intact insecticidal containing microcapsule.

Of these types of adjuvants, most are introduced into the core of the microparticle by incorporation into the dispersed phase. Only the last type of adjuvant is incorporated in the continuous aqueous phase. Representative of the first type of adjuvant is a low molecular weight mono- or poly-carboxylic acid ester or amide which is highly lipophilic, but which also has some limited hydrophilic character. Adjuvents of this type useful in the present invention include triethyl citrate acetate, diethyl succinate, triethylcitrate, diethyl-2-acetylsuccinate, diethyl-2-acetylglutarate, glyceryl triacetate, glyceryl tributyrate and N-methyl gamma pyrrolidinone.

Various functions of the first type adjuvant are described above. A specific function of that adjuvant is solubilizing a low density constituent compound such as a mineral oil, light liquid petrolatum, long chain alkane or crop oils such as corn or safflower oil. The latter are used to alter the buoyant density of the intact microparticle to make the microparticulate material buoyant in a pond or marsh. These "floatable forms" of microparticles are particularly useful against surface and filter feeding insects such as mosquitoes. By concentrating insecticidal toxins at the surface in the form of floating toxin microparticles which are ingested with food by insects, much lesser mounts of toxin are required to control an insect population than would be needed to produce an insecticidal concentration of toxin in an entire pond or marsh.

For some applications of insecticidal toxin-containing microcapsules, such as for the control of leaf eating insects, it may be desirable to produce particularly robust or reinforced microcapsules to facilitate application with mechanical or handheld sprayers in the field or forest. In such instances, thick walled or reinforced microcapsules may be preferred for "fogging" orchards and the like. By the incorporation of a reinforcing adjuvant, such as ethylcellulose, the mechanical strength of insecticidal toxin containing micro-particles may be increased.

In a further embodiment of the present invention, the Lewis acid and Lewis base reactants are utilized in the form of their salts in the process for producing the microparticles of U.S. 3,959,457, rather than in the form of their free acid and base form. The salt forms of the Lewis acid and Lewis base react to form a new anisotropic salt of the acid and base and a neutral salt of the inorganic cation and anion in what is sometimes termed a "double decomposition" or "salt exchange" reaction.

In this embodiment of the invention, two pairs of oppositely charged species (namely, the salt form of a Lewis acid moiety and the salt form of a Lewis base moiety), interact to generate two new pairs of oppositely charged ions. One new pair of oppositely charged ions (e.g., the benzalkonium salt of carboxymethylcellulose or benzalkoniummethylcellulose carboxylate) is poorly soluble at the phase interface in the emulsion reaction medium and precipitates to form the wall of the microcapsule. The other new pair of oppositely charged ions (e.g., sodium chloride) is typically fairly water soluble and readily dissolves in the aqueous medium. Thus, the salt ion of the Lewis acid salt reacts with the salt ion of the Lewis base salt to form a new anisotropic salt of the acid and base and a neutral salt of the inorganic cation and anion in a "double decomposition" reaction. Useful salts in the practice of the present invention are those which react to form an anisotropic wall-forming salt film according to U.S. 3,959,457 and an organic or inorganic neutral salt soluble in the aqueous phase of the emulsion. Broadly, any of the core substances mentioned in U.S. Patent 3,959,457 may be encapsulated by this "double decomposition" reaction process, as well as the proteinaceous core substances described herein. The process provides an improved method for employing more completely solubilized reactants, especially the difficult-to-solubilize high molecular weight Lewis acids utilized, which typically exhibit quite limited solubility in aqueous solution in the form of the free (unionized) acids. Salts of macromolecular polyanions such as sodium carboxymethylcellulose or sodium polyacrylate are much more water soluble than are the corresponding macromolecules as free acids. Advantages of the use of salts of acids accrue from the greater ease in preparing manufacturing solutions, as well as the greater range of reactant concentrations available and the consequent greater opportunity to control characteristics (e.g. thickness) of the resultant microcapsule walls. In order to obtain these advantages, as well as stable anisotropic salt films, salts of both the Lewis acid and base reactants are employed, as the combination of a free acid or free base with a salt reactant tends to provide products unsuitable for use is encapsulating films in accordance with the invention.

Microcapsules made from Lewis acid and base reactants in their salt forms may be readily made in the range of sizes from below 1 $\mu$m to above 1000 $\mu$m by modifying dispersion techniques (i.e., intensity and/or duration) to produce emulsion droplets of the appropriate size. These new microcapsules have very high internal to wall volume ratios, thus high core loading fractions may be attained. Electron micrographs show the walls can be made only a fraction of a $\mu$m thick with the central volume clearly demarked from the wall proper. Furthermore, use of the wall-forming components in salt form can confer advantageous properties on the resultant microcapsules which are not present in the microparticles described in U.S. Patent 3,959,457. For example, microcapsules made by reaction of benzalkonium chloride and sodium carboxymethylcellulose, as described above, are markedly more stable than are those derived from piperazine and arabic acid, according to U.S. Patent 3,959,457. Microcapsules with walls of benzalkonium methylcellulose carboxylate are stable at 50°C (122°F) for many hours, while microcapsules with walls of piperazine arabate degrade after a few minutes at that temperature.

Other salt-form microcapsules with differing wall components may be made by employing as Lewis base and Lewis acid salt forms cetylpyridinium chloride, or the sodium salt of a poly-acrylic acid or the sodium salt of polyoxyethylene cross-linked with poly-acrylic acid (e.g. Carbopol® 934, a product of B. F. Goodrich), Furthermore, microcapsules may also be formed by substituting both the benzalkonium chloride and sodium carboxymethylcellulose with cetylpyridinium chloride and the sodium slat of a poly-acrylic acid or the sodium salt of poly-oxyethylene cross-linked with poly-acrylic acid, respectively. Preferably, the acid and base functional groups of the Lewis acids and bases are substantially completely substituted respectively with the salt-forming cations and anions.

In an additional embodiment of the present invention, one or more wall-forming or core-forming adjuvants are included in the emulsion reaction mixture to enhance wall and/or core substance formation in reactions employing either Lewis acids and bases or salts thereof as wall-forming reactants, and employing proteinaceous or non-proteinaceous materials as a core substance.

In accordance with the present invention, the adjuvant or adjuvants are dissolved in the aqueous solution or in the non-aqueous solvent or both, prior to combining the aqueous and non-aqueous solutions and forming the emulsion. Selected adjuvants must, of course, be substantially non-reactive with other components in the reaction medium, including the core substance and must also be substantially soluble in the appropriate solvent phase.

Adjuvants are of two types, wall-forming and core-forming. Core-forming adjuvants are understood to be entirely within the core of the finished microcapsule. Core-forming adjuvants are typically polyoxyethylenepolyoxypropylene copolymers or block copolymers thereof. They are referred to herein as "poloxamers" and are added to the non-aqueous, slightly polar organic phase before the emulsification step.

Wall-forming adjuvants contribute to both wall and core formation. They combine in their molecular structures either acidic or basic functions together with polyether chains of varying length. Wall-forming adjuvants which combine acidic functional groups with ether chains are exemplified by high molecular weight polymers of acrylic acid cross-linked with a polyalkenyl polyether; such adjuvants are referred to herein as "carbomers". Wall-forming adjuvants which combine basic functional groups with polyether chains are exemplified by ethylene oxide adducts of ethylenediamine, and are referred to herein as "tetronomers".

Both carbomers and tetronomers react with ionizable species at the inter-phase boundary during the wall-forming process to form salts which become integral components of the ionic salt wall structure of the microcapsule. The polyether chains of carbomers and tetronomers are thought to project inwardly from the inner surface of the microcapsule wall for short distances, into what might be described as a core space.

In practice, carbomers are dissolved in the aqueous phase of the manufacturing system. Tetronomers are dissolved in the non-aqueous, slightly polar organic phase. Tetronomers and carbomers fulfill a primary function as wall-forming materials and also act as adjuvants.

The wall-forming and core-forming adjuvant-containing microparticles of the present invention are physically more robust and are able to withstand greater mechanical and thermal stress than non-adjuvant containing microparticles. For example, when warmed in a water suspension of approximately 40°C, the non-adjuvant containing microparticles of U.S. Patent 3,959,457 will readily dissolve. In contrast, adjuvant containing microparticles of the present invention, comprised of essentially the same Lewis acid/Lewis base combinations, are stable under these conditions and only begin to dissolve at temperature near 80°C. Similarly, continued agitation of non-adjuvant containing microparticles can result in their rupture while adjuvant containing microparticles are able to withstand vigorous shaking for extended periods.

Furthermore, even apart from their use in encapsulated protein systems adjuvant-modified microparticles may facilitate encapsulation of a wider range and/or greater amount of core materials. Many substances are more readily and more extensively soluble in adjuvant-containing, non-aqueous manufacturing solvents than in those same solvents without the adjuvant. For example, piperazine arabate walled microcapsules containing mineral oil as a core material may be seen to begin to coalesce within minutes after manufacture and degrade extensively and separate into aqueous and oily layers, free of capsular material, within hours after manufacture. However, the addition of a core-forming adjuvant (such as polyoxyethylene-polyoxypropylene block polymer, Pluronic® F68) to the organic phase before the emulsification step results in piperazine arabate walled microcapsules of mineral oil which remain stable for months. The microencapsulation of mineral oil, through the use of an adjuvant, demonstrates the encapsulation of a wider range of core materials than had been possible without the use of an adjuvant.

Furthermore, the use of a core-forming adjuvant enables greater concentrations of relatively polar core materials, such as acetanilide, to be dissolved in the organic manufacturing solvent. Thus, more core material can be encapsulated than is possible without the adjuvant.

Additionally, acidic drugs, such as certain non-steroidial anti-inflammatory agents, exemplified by salicylic acid and ibuprofen, which interfere with wall formation in non-adjuvant containing microparticles, may successfully be encased in adjuvant-containing microparticles.

Release of core materials (e.g., pharmaceuticals) from the adjuvant-containing microparticles of the present invention can be more extensively controlled than can release from non-adjuvant containing microparticles made according to the teachings of U.S. Patent 3,959,457. For example, in the absence of an adjuvant, a quite water soluble substance, such as the model drug acetanilide, may be released from microcapsules into surrounding aqueous medium essentially completely within the space of an hour. However, the addition of a small amount of a core-forming adjuvant can increase the duration of the period of release by at least a factor of 10. In effect, the core-forming adjuvant modifies the release properties of the micro-capsular system so that, while it retains the diffusional barrier provided by the capsular wall, release of the drug from the adjuvant-containing system is influenced by the partitioning equilibrium between the adjuvant and the small volume of water which diffuses into the capsule. Thus, the proportion of adjuvant to active core material (e.g., acetanilide) in the microcapsule formulation and the rate of release of active core material are inversely proportional, all other factors being held constant.

Not only may the ratio of core-forming adjuvant to active core material be modified, but the wide range of compositions of core-forming adjuvants with markedly different partitioning characteristics for the same substance allow a second means of controlling rates of release of the active core component.

Independently, the wall-forming adjuvants, by virtue of the differences in ionic lattice spacing of the microcapsules they provide, afford yet another means of controlling rates of release of active core components. It is these three variables, the ratio of core-forming adjuvant to active core component, the range of partitioning coefficient of the active core component between core-forming adjuvants and water, and the lattice structure variation available from wall-forming adjuvants which provide extensive control of the release rate for encapsulated substances.

Thus, the improved controlled release characteristics of adjuvant-modified microparticles, coupled with the improved capacity of such microparticles, allow formulation of microparticles capable of uniform sustained release of core components over a greatly extended period of time.

The products of the inventions are versatile, readily prepared delivery systems which do not tend to agglomerate, in contrast to prior art microparticulate material. Further, for intravenous use, the microparticulate material of the present invention resists capture by the reticulo-endothelial system, a common problem with known prior art delivery systems based on microparticulate material. As a result, good blood clearance and tissue distribution properties are obtained with microparticles according to the present invention in such applications.

EXAMPLES

General Procedures for Forming Microcapsules

In all instances where an aqueous solution is utilized as the continuous phase for the dispersion or emulsification of a second solution of materials dissolved in an organic solvent, it is preferred, but not essential, that the organic solvent be slowly and steadily added to the aqueous solution over a period of approximately 30 seconds. In all instances, solutions are prepared and reactions take place at room temperature, unless otherwise stated. Any of several means to disperse or emulsify the organic solution in the aqueous medium may be employed including:

a. vigorously stirring the solution with a magnetically driven stirring bar at a nominal sheer rate of 700 or more cm/s;

b. vigorously mixing the solution with a multi-orifice axial turbine (such as a Brinkmann homogenizer PT10/35 and generator PST/10, Brinkmann Instruments, Westbury, N.Y.) at a nominal setting of 5; or

c. vigorously agitating the solutions with an ultrasonic probe (such as Heat Systems model W185D, Ultrasonics, Inc., Plainview, N.Y.) at a nominal output of 100 W.

By increasing or decreasing length of time or vigor of emulsification, droplet size (and resulting microparticle size) may be controlled. Typically, microparticle size ranges from 3 to 150 $\mu$m, although for specific applications larger or smaller particles may be desirable.

After formation of the core substance-containing microparticles, the newly formed microcapsules may be alowed to settle to the bottom of the reaction container whereupon the non-emulsified, non-aqueous organic phase is separated from the microcapsules and the aqueous phase. However, the separation step is preferably accelerated by centrifugation at approximately 10,000 gravity minutes.

Example I: Method of Making MicrocapsuleProducts Containing Proteinaceous Core Substance

An aqueous solution of arabic acid was prepared by adding to 1 g of arabic acid, enough water to make 10ml. Typically, the arabic acid is first wetted with a small amount of alcohol to assist in solubilizing the otherwise slowly solubilized Lewis acid. A non-aqueous solution was also prepared by adding anhydrous piperazine (in an amount stoichiometrically equivalent to the arabic acid), 0.005 g of somatotrophic hormone or somatotrophin (growth hormone), in enough butyrolactone to make 10ml of solution.

The aqueous and non-aqueous solutions were then combined in a container and continuously agitated for approximately one minute, to produce an emulsion of organic droplets, approximately 5 $\mu$m in diameter, dispersed in and surrounded by continuous phase aqueous solution.

After agitation, the mixture was centrifuged (10,000 gravity minutes) and the supernatant fluid was removed from the viscid volume of packed newly formed microcapsules. The microcapsules were washed of manufacturing fluids and unreacted or excess reaction components by repeatedly suspending them in an equal volume of water. The suspended microparticles were separated by centrifuging (10,000 gravity minutes) and removing the supernatant. The resultant product was a flowable concentrate of microparticulate material comprising microcapsules consisting of shell-like films surrounding the protein core material.

Example II: Method of Making MicrocapsuleProducts Containing Proteinaceous Core Substance

An aqueous solution of arabic acid was prepared by adding to 1 g of arabic acid, enough water to make 10ml. A first non-aqueuous solution was prepared by adding anhydrous piperazine (in an amount stochiometrically equivalent to the arabic acid) in enough dichloromethane to equal 2 ml. A second non-aqueous solution was also prepared by adding 0.010 g of somatotropic hormone or somatotropin (growth hormone) to enough N-methylformamide to make 8 ml.

The first and second non-aqueuous solutions were then combined and dispersed into the aqueous solution. The aqueous and non-aqueous mixture was then continuously agitated for approximately one minute, to produce an emulsion of organic droplets, approximately 5 $\mu$m in diameter, dispersed in and surrounded by continuous phase aqueous solution.

After agitation, the mixture was allowed to stand undisturbed for about 10 minutes and the supernatant suspension was removed from the small volume of dichloromethane which separated. Residual dichloromethane was removed by evaporation. The mixture was centrifuged (10,000 gravity minutes) and the supernatant fluid was removed from the highly viscid volume of packed newly formed microcapsules. The microcapsules were washed of manufacturing fluids and unreacted or excess reaction components, by repeatedly suspending them in an equal volume of water separating the suspended microparticles by centrifuging (10,000 gravity minutes), and removing the supernatant. The resulting product was a flowable concentrate of microparticulate material comprising microcapsules consisting of shell-like films surrounding the protein core material.

Example III: Method of Making MicrocapsuleProducts Containing Proteinaceous Core Substance

A solution of arabic acid was prepared by adding to 1 g of arabic acid enough water to make 10 ml. A first non-aqueous solution was prepared by adding to anhydrous piperazine (in an amount stoichiometrically equivalent to the arabic acid) in enough dichloromethane to make 2 ml. A second non-aqueous solution was also prepared by adding 0.01 g of insulin to enough N-methylformamide to make 8 ml.

The first and second non-aqueous solutions were then combined and then dispersed into the aqueous solution with continuous and vigorous agitation for approximately one minute to produce an emulsion of organic droplets approximately 5 $\mu$m in diameter, dispersed in and surrounded by a continuous aqueous phase solution. After agitation, the mixture was allowed to stand undisturbed for about 10 minutes and the supernatant suspension was removed from the small volume of dichloromethane which separated. Residual dichloromethane was removed by evaporation. The mixture was centrifuged (10,000 gravity minutes) and the supernatant fluid was removed from the highly viscid volume of packed newly formed microcapsules. The microcapsules were washed of manufacturing fluids and unreacted or excess reaction components, by repeatedly suspending them in an equal volume of water, separating the suspended microparticles by centrifuging (10,000 gravity minutes), and removing the supernatant. The resulting product was a flowable concentrate of micro-particulate suspension comprising microcapsules consisting of shell-like films surrounding protein core material.

Example IV: General Method of Making Microcapsule Products Containing Bacillus Thuringiensis Insecticidal Toxins

An aqueous solution of arabic acid was prepared by adding to 1 g of arabic acid, enough water to make 10ml. A non-aqueous solution was also prepared by adding anhydrous piperazine (in an amount stoichiometrically equivalent to the arabic acid), to enough dichloromethane to make 10ml of solution. Suspended in the dichloromethane solution was 0.010 g of finely divided Bt insecticidal toxin.

The aqueous and non-aqueous solutions were then combined in a container and continuously agitated for approximately one minute, to produce an emulsion of organic droplets, approximately 3 to 150 $\mu$m in diameter, dispersed in and surrounded by continuous phase aqueous solution.

After agitation, the emulsion was centrifuged at approximately 10,000 gravity minutes to separate the aqueous and non-aqueous phases as layers and to settle the newly formed microcapsules to the bottom of the container. The aqueous phase was then removed along with any residual clear, non-aqueous organic phase. Unreacted or excess reaction components were then removed by adding an equal amount of water to the microcapsules and subsequent removal of the added water. Residual dichloromethane was removed by evaporation upon exposure of the aqueous suspension of the microcapsules to the atmosphere. Upon centrifugation and removal of supernatant water, the microparticulate material was rendered as a flowable concentrate of microcapsules. Upon drying, microparticulate material comprising microcapsules consisting of shell-like films surrounded the core material including the Bacillus thuringiensis toxin.

Example V: General Method of Making Buoyant Microcapsule Products Containing Bacillus Thuringiensis Insecticidal Toxins

An aqueous solution of arabic acid was prepared by adding to 1 g of arabic acid, enough water to make 10 ml. A non-aqueous solution was also prepared by adding to anhydrous piperazine (in an amount stoichiometrically equivalent to the arabic acid), to enough dichloromethane to make 10ml of solution. Then added to the dichloromethane solution with mixing was 5.0 ml of light liquid petrolatum (mineral oil) and 3.0 ml of triethylcitrate acetate. Suspended in this mixture was 0.010 g of finely divided crystal toxin of Bacillus thuringiensis.

The aqueous and non-aqueous solutions were combined in a container and continuously agitated for approximately one minute, to produce an emulsion of organic droplets, approximately 3 to 150 $\mu$m in diameter, dispersed in and surrounded by continuous phase aqueous solution.

After agitation, the emulsion was centrifuged at approximately 10,000 gravity minutes to separate the aqueous and non-aqueous phases as layers and to settle the newly formed microcapsules to the bottom of the container. The aqueous phase was then removed along with any residual clear, non-aqueous organic phase. Unreacted or excess reaction components were then removed by adding an equal amount of water

11

to the micro-capsules and subsequent removal of the added water. Residual dichloromethane was removed by evaporation upon exposure of the aqueous suspension of the micro-capsules to the atmosphere. Upon centrifugation and removal of supernatant water, the microparticulate material was rendered as a flowable concentrate of microcapsules. Upon drying, microparticulate material comprising microcapsules consisting of shell-like films surrounded the core material including the Bacillus thuringiensis toxin.

Example VI: General Method of Making Adhesive Microcapsule Products Containing Bacillus Thuringiensis Insecticidal Toxins

An aqueous solution of arabic acid was prepared by adding to 1 g of arabic acid, enough water to make 10ml. A non-aqueous solution was also prepared by adding anhydrous piperazine (in an amount stoichiometrically equivalent to the arabic acid) enough dichloromethane to make 10 ml of solution. Then, suspended in the dichloromethane solution was 0.010 g of finely divided insecticidal toxin derived from Bacillus thuringiensis. The aqueous and non-aqueous solutions were then combined in a container and continuously agitated for approximately on minute, to produce an emulsion of organic droplets, approximately 3 to 150 $\mu$m in diameter, dispersed in and surrounded by continuous phase aqueous solution.

After agitation, the emulsion was centrifuged at approximately 10,000 gravity minutes to separate the aqueous and non-aqueous phases as layers and to settle the newly formed microcapsules to the bottom of the container. The aqueous phase was then removed along with any residual clear, non-aqueous organic phase. Unreacted or excess reaction components were then removed by adding an equal amount of water to the microcapsules and subsequent removal of the added water. Residual dichloromethane was removed by evaporation upon exposure of the aqueous suspension of the microcapsules to the atmosphere. Upon centrifugation and removal of supernatant water, the microparticulate material was rendered as a flowable concentrate of microcapsules. Upon drying, microparticulate material comprising microcapsules consisting of shell-like films surrounded the core material including the Bacillus thuringiensis toxin.

The toxin containing microparticles were then dispersed in a nominally 10% aqueous solution of arabic acid, gum arabic or other acidic or neutral macromolecule. Alternatively, adhesive microparticles may effectively be produced by the use of an excess amount of the acidic or neutral macromolecule in the aqueous phase prior to combining the non-aqueous and aqueous phases and omitting the wash steps wherein excess and unreacted polymeric material is removed by addition of water, resuspension of the microcapsule layer, centrifuging, and separation of the new aqueous layer.

Example VII: General Method of Making Buoyant Microcapsule Products Containing Bacillus Thuringiensis Insecticidal Toxins and Sunscreen

An aqueous solution of arabic acid was prepared by adding to 1 g of arabic acid enough water to make 10ml. A non-aqueous solution was also prepared by adding anhydrous piperazine (in an amount stoichiometrically equivalent to the arabic acid) to enough dichloromethane to make 10ml of solution.

Then added to the dichloromethane solution with mixing was 5.0ml of light liquid petrolatum (mineral oil), 3ml of triethylcitrate acetate and 0.10 g of phenyl salicylate. Suspended in this mixture was 0.01 g of finely divided crystal toxin from Bacillus thuringiensis.

The aqueous and non-aqueous solutions were combined in a container and continuously agitated for approximately one minute to produce an emulsion of organic droplets approximately 3 to 150 $\mu$m in diameter dispersed in and surrounded by a continuous aqueous phase solution.

The emulsion was centrifuged at approximately 10,000 gravity minutes to separate the aqueous and non-aqueous phases as layers and to settle the newly formed microcapsules to the bottom of the container. The aqueous phase was then removed along with any residual clear, non-aqueous organic phase. Unreacted or excess reaction components were then removed by adding an equal amount of water to the microcapsules and subsequent removal of the added water. Residual dichloromethane was removed by evaporation upon exposure of the aqueous suspension of the microcapsules to the atmosphere. Upon centrifugation and removal of supernatant water, the microparticulate material was rendered as a flowable concentrate of microcapsules. Upon drying, microparticulate material comprising microcapsules consisting of shell-like films surrounded the core material including the Bacillus thuringiensis toxin.

Example VIII: General Method of Making Reinforced Buoyant Microcapsule Products Containing Bacillus Thuringiensis Insecticide Toxins and Sunscreen

An aqueous solution of arabic acid was prepared by adding to 1 g of arabic acid enough water to make 10ml. A non-aqueous solution was also prepared by adding anhydrous piperazine (in an amount stoichiometrically equivalent to the arabic acid) to enough dichloromethane to make 10ml of solution. Then added to the dichloromethane solution with mixing was 5.0 ml of light liquid petrolatum (mineral oil), 3ml of triethylcitrate acetate, 0.10 g of phenyl salicylate and 0.1 g of ethylcellulose. Suspended in this mixture was 0.01 g of finely divided crystal toxin of Bacillus thuringiensis.

The aqueous and non-aqueous solutions were combined in a container and continuously agitated for approximately one minute to produce an emulsion of organic droplets approximately 3 to 150 $\mu$m in diameter, dispersed in and surrounded by continuous aqueous phase solution.

The emulsion was centrifuged at approximately 10,000 gravity minutes to separate the aqueous and non-aqueous phases as layers and to settle the newly formed microcapsules to the bottom of the container. The aqueous phase was then removed along with any residual clear, non-aqueous phase. Unreacted or excess reaction components were then removed by adding an equal amount of water to the microcapsules and subsequent removal of the added water. Residual dichloromethane was removed by evaporation upon exposure of the aqueous suspension of the microcapsules to the atmosphere. Upon centrifugation and removal of supernatant water, the microparticulate material was rendered as a flowable concentrate of microcapsules. Upon drying, micropartics material comprising microcapsules consisting of shell-like films surrounded the core material including the Bacillus thuringiensis toxin.

Example IX: General Procedure of Preparing Microcapsules from a Lewis Acid Salt and a Lewis Base Salt

An aqueous solution of sodium carboxymethylcellulose was prepared by adding to 0.01 g of sodium carboxymethyl cellulose enough water to make 10ml. A non-aqueous solution was also prepared by adding (in an amount stoichiometrically equivalent to the sodium carboxymethyl cellulose), anhydrous benzalkonium chloride and 1.0 g of a core material (acetanilide) in enough dichloromethane to make 10ml of solution.

The aqueous and non-aqueous solutions were then combined in a container and continuously agitated for approximately one minute, to produce an emulsion of organic droplets, approximately 5 $\mu$m in diameter, dispersed in and surrounded by continuous phase aqueous solution.

Upon standing after agitation the newly formed microcapsules were allowed to separate from the bulk of the aqueous phase and settle to the bottom of the container. The separation was speeded by centrifugation. The clear supernatant aqueous phase was then removed along with any residual clear non-aqueous organic phase which may have separated. Unreacted or excess reaction components were then removed by dispersing the microcapsules in an equal amount of water and again removing the supernatant liquid from the packed microcapsules. Residual dichloromethane was removed by evaporation upon exposure of the microcapsules to the atmosphere. The product comprised a flowable concentrate of microparticulate material consisting of microcapsules of shell-like films surrounding the (acetanilide) core material.

Example X: General Method of Making Microcapsule Products Containing Core-Forming Adjuvant Materials

An aqueous solution of arabic acid was prepared by adding to 1 g of arabic acid, enough water to make 10ml. Typically, the arabic acid is first wetted with a small amount of alcohol to assist in solubilizing the otherwise slowly solubilized Lewis acid. A non-aqueous solution was also prepared by adding anhydrous piperazine (in an amount stoichiometrically equivalent to the arabic acid), 1.0 g of a core material (acetanilide), and 1.0 g of poloxamer (polyoxyethelene-polyoxypropylene block copolymer, such as Pluronic® F68, a product of BASF-Wyandotte Corp., Wyandotte, Illinois), to enough dichloromethane to make 10ml of solution.

The aqueous and non-aqueous solutions were then combined in a container and continuously agitated for approximately one minute, to produce an emulsion or organic droplets, approximately 5 $\mu$m in diameter, dispersed in and surrounded by continuous phase aqueous solution.

Upon standing after agitation, the non-aqueous organic phase was allowed to separate from the aqueous phase. The essentially-clear, non-aqueous organic phase was then removed from the aqueous phase containing the microcapsules. The milk-like suspension of newly formed microcapsules settled to the bottom of the container. Unreacted or excess reaction components were then removed by adding an equal amount of water to the microcapsules and subsequent removal of the added water. Residual dichloromethane was removed by evaporation upon exposure of the microcapsules to the atmosphere. The suspension of dichloromethane-free microcapsules was centrifuged to produce a flowable concentrate of microparticulate material comprised of microcapsules consisting of shell-like films surrounding the core material (acetanilide) and the adjuvant.

13

Example XI: General Method of Making Microcapsule Products Containing Wall-Forming Adjuvant Materials

Microparticulate material prepared by the process of the present invention can be prepared by omitting either the conventional Lewis acids or the conventional Lewis bases. In these products, polymers of acrylic acid (providing an acidic moiety) cross-linked polyalkenyl polyethers (providing an adjuvant moiety) may perform the function of the Lewis acid. The Lewis bases may also be substituted for by the use of polyoxyethylene adducts (the adjuvant moiety) of ethylenediamine (the Lewis base moiety).

In both cases the polyether part of the Lewis acid or Lewis base molecule functions as an adjuvant in accordance with the present invention. For example, an aqueous solution of a carbomer was prepared by adding to 0.1 g of finely divided polyacrylic acid cross-linked with polyalkenyl polyethers such as Carbomer®-68, (a product of Rohm and Haas Company, Philadelphia, PA) enough water to make 10ml. A non-aqueous solution was also prepared by adding a stoichiometric amount of anhydrous piperazine and 1.0 g of a core material (acetanilide) to enough dichloromethane to make 10ml.

The aqueous and non-aqueous solutions were then combined in a container and continuously agitated for approximately 1 minute to produce an emulsion of organic droplets of approximately 5 $\mu$m in diameter in continuous phase comprising the aqueous solution.

The resulting product was handled as previously described, yielding a flowable microparticle material comprised of microcapsules consisting of shell-like films surrounding the core material.

Alternatively, for example, an aqueous solution of arabic acid was prepared by adding to 1.0 g of arabic acid enough water to make 10 ml. A non-aqueous solution was also prepared by adding 1.0 g of core material (acetanilide) and 1.0 g of a poly-oxyethylene adduct of ethylenediamine (such as Tetronic® 702, a product of BASF-Wyandotte Corp., Wyandotte, Illinois), to enough dichloromethane to make 10 ml.

The aqueous and non-aqueous solutions were then combined in a container and continuously agitated for a minute to produce an emulsion of organic droplets, approximately 5 $\mu$m in diameter, in an aqueous continuous phase. The resulting product was handled as previously described, to yield a flowable microparticulate material consisting of shell-like films surrounding the core material.

**Claims**

1.  A delivery system for the gradual release of a core substance from a microcapsule comprising a core and a microcapsule wall of an anisotropic salt film which is the reaction product of
    (a) a Lewis base or a salt thereof having a basic strength ($pK_a$) of from about 7 to 12 and reacting at the interfaces of the organic and aqueous phases of an emulsion through dipole and/or ionic bonding, in a slightly polar organic solvent for the base or salt and an optional adjuvant, and
    (b) a polyfunctional partially hydrophilic, partially lipophilic Lewis acid or salt thereof having reactive or cross-linking sites spaced about a distance equivalent to a normal carbon chain containing at least 20 and no more than 360 carbon atoms in an aqueous solution or suspension and an optional adjuvant,
    characterized in that the core substance is either a pharmaceutical material or a proteinaceous material comprising a biologically-active peptide or polypeptide containing from 14 to 100 amino acids, and the slightly polar organic solvent is a non-denaturing solvent or solvent complex sufficiently polar to anisotropically orient the salt-forming components and stabilize the product salt film against hydration in the aqueous phase and capable of solubilizing or suspending the proteinaceous material without deactivation thereof.

2.  The delivery system of claim 1 wherein the Lewis base is selected from the group consisting of monofunctional amines, hexylamine, isopentylamine, N-methylpiperidine, piperidine, difunctional amines, dimethylethylenediamine, hexanediamine, piperazine, triethylenediamine, ethylenediamine, polyfunctional amines, hexamethylrosanilinium chloride, rosanilium chloride, tetramethylrosanilium chloride, melamine, tetraethylenepentamine and triethylenetetramine, and/or the Lewis acid is selected from the group consisting of agar, acacia gum, arabic acid, carboxymethylcellulose, ghatti gum, guar gum, methylcellulose, oxidized cellulose, pectin, tragacanth and polyethylene glycol.

3.  The delivery system of claim 1 or claim 2 wherein the proteinaceous material is an insecticidal toxin.

4.  The delivery system of claim 3 wherein the insecticidal toxin is derived from Bacillus thuringiensis.

5.  The delivery system of claim 1 or claim 2 wherein the proteinaceous material is insulin, glucagon or

EP 0 299 205 B1

growth hormone.

6. The delivery system of any of claims 1 to 5 wherein the slightly polar organic solvent is selected from the group consisting of bromoform, chloroform, dichloromethane, dichloroethane, diethyl ether, diisopropyl ether, methyl ethyl ketone and nitrobenzene.

7. The delivery system of any of claims 1 to 6 wherein the non-denaturing solvent is a cyclic ester or a cyclic or linear amide having a Hildebrand solubility parameter of approximately 23 to approximately 32 MPa $^{1/2}$.

8. The delivery system of claim 7 wherein said cyclic ester is butyrolactone, valerolactone or caprolactone; said cyclic amide is N-methyl-gamma-pyrrolidone;and said linear amide is N-methylformamide, N-methylacetamide, N,N-dimethylformamide, N,N-dimethylacetamide or N-ethylacetamide.

9. The delivery system of any of claims 1 to 8 wherein said non-denaturing solvent or solvent complex comprises a first non-denaturing, solubilizing solvent for said proteinaceous material and a second solvent selected from the group consisting of chloroform, dichloromethane, dichloroethane and methyl ethyl ketone.

10. The delivery system of any of claims 1 to 9 wherein the basic and acidic functional moieties of said Lewis acid or Lewis base are reactants substantially completely substituted with a salt-forming ion and react to form said anisotropic salt film and a neutral salt.

11. The delivery system of any of claims 1 to 10 wherein said Lewis acid has a molecular weight of at least about 12,000.

12. The delivery system of any of claims 1 to 11 wherein the microcapsule wall or core further includes a toxin-promoting adjuvant comprising a sunscreen, a buoying agent, a reinforcing agent or an adhesive.

13. The delivery system of claim 12 wherein the non-denaturing solvent further includes an ester or amide which promotes encapsulation of a sunscreen or a buoying agent comprising a low density constituent.

14. The delivery system of any of claims 1 to 13 wherein the microcapsule wall or core substance further includes an adjuvant comprising a high molecular weight polymer of acrylic acid cross-linked with a polyalkenyl polyether, a polyoxyethylene-polyoxypropylene or a block copolymer thereof or a polyoxyethylene adduct of ethylenediamine.

15. A method for the production of the delivery system of any of claims 1 to 14 for the gradual release of a core substance from a microcapsule comprising a core and a microcapsule wall of an anisotropic salt film, characterized in that the delivery system is produced by reacting, in the form of an emulsion,
    (a) a first reaction system comprising
        (i) a first reactant mixture of a Lewis base or a salt thereof having a basic strength ($pK_a$) of from about 7 to 12 and reacting at the interfaces of the organic and aqueous phases of an emulsion through dipole and/or ionic bonding, in a slightly polar organic solvent for the base or salt, said slightly polar organic solvent being a non-denaturing solvent or solvent complex sufficiently polar to anisotropically orient the salt-forming components and stabilize the product salt film against hydration in the aqueous phase and capable of solubilizing or suspending the core substance without deactivation thereof, an optional adjuvant and a core substance, said core substance being either a pharmaceutical material or a proteinaceous material comprising a biologically-active peptide or polypeptide containing from 14 to 100 amino acids and being solubilizable or suspendable in said organic solvent, with
        (ii) a second reactant mixture of a polyfunctional partially hydrophilic, partially lipophilic Lewis acid or salt thereof having reactive or cross-linking sites spaced about a distance equivalent to a normal carbon chain containing at least 20 and no more than 360 carbon atoms in an aqueous solution or suspension and an optional adjuvant; or
    (b) a second reaction system comprising
        (i) a first reactant mixture comprising a salt of said Lewis base in said slightly organic solvent for the base, an optional adjuvant and said core substance, with

15

(ii) a second reactant mixture comprising a salt of said polyfunctional Lewis acid in an aqueous solution or suspension and an optional adjuvant; or

(c) a third reaction system comprising

(i) a first reactant mixture comprising said Lewis base or a salt thereof in said slightly organic solvent for the base and said core substance, with

(ii) a second reactant mixture comprising said polyfunctional Lewis acid or a salt thereof in an aqueous solution or suspension, and with

(iii) a third reactant mixture comprising a wall-forming or core-forming adjuvant or a combination thereof;

to form a core substance microencapsulated in an anisotropic salt film through which the core substance is gradually permeable.

16. The method of claim 15 wherein the Lewis base is selected from the group consisting of monofunctional amines, hexylamine, isopentylamine, N-methylpiperidine, piperidine, difunctional amines, dimethylethylenediamine, hexanediamine, piperazine, triethylenediamine, ethylenediamine, polyfunctional amines, hexamethylrosanilium chloride, rosanilium chloride, tetramethylrosanilium chloride, melamine, tetraethylenepentamine and triethylenetetramine, and/or the Lewis acid is selected from the group consisting of agar, acacia gum, arabic acid, carboxymethylcellulose, ghatti gum, guar gum, methylcellulose, oxidized cellulose, pectin, tragacanth and polyethylene glycol.

17. The method of claim 15 and claim 16 wherein the slightly polar organic solvent is selected from the group consisting of bromoform, chloroform, dichloromethane, dichloroethane, diethyl ether, diisopropyl ether, methyl ethyl ketone and nitrobenzene.

18. The method of any of claims 15 to 17 wherein wherein the proteinaceous material is an insecticidal toxin.

19. The method of claim 18 wherein the insecticidal toxin is derived from Bacillus thuringiensis.

20. The method of any of claims 15 to 19 wherein the Lewis base or salt thereof is polyfunctional.

21. The method of any of claims 15 to 20 wherein the delivery system is produced by reacting the first and second reactant mixtures of claim 15 (b).

22. The method of claim 21 wherein said Lewis base salt is benzalkonium chloride or cetylpyridinium chloride, and wherein said polyfunctional Lewis acid salt is sodium carboxymethylcellulose, a sodium salt of a polyacrylic acid or a sodium salt of a polyoxyethylene cross-linked polyacrylic acid.

23. The method of any of claims 15 to 20 wherein the delivery system is produced by reacting the first, second and third reactant mixtures of claim 15 (c).

24. The method of claim 23 wherein the third reactant of claim 15 (c) (iii) is a wall-forming adjuvant.

25. The method of claim 23 wherein the third reactant of claim 15 (c) (iii) is a core-forming adjuvant.

26. The method of any of claims 23 to 25 wherein said wall-forming or core-forming adjuvant is a high molecular weight polymer of acrylic acid cross-linked with a polyalkenyl polyether, a polyoxyethylene-polyoxypropylene or a block copolymer thereof or a polyoxyethylene adduct of ethylenediamine.

**Revendications**

1. Système débiteur ou distributeur pour la libération graduelle d'une substance formant le noyau d'une microcapsule comprenant un noyau et une paroi de microcapsule faite d'un film d'un sel anisotrope, qui est le produit de la réaction de

(a) une base de Lewis ou un sel de cette base, fortement basique (pKa) d'environ 7 à 12 et réagissant aux interfaces des phases organique et aqueuse d'une émulsion par liaison dipolaire et/ou ionique, dans un solvant de la base ou du sel légèrement polaire et un adjuvant facultatif et

(b) un acide de Lewis polyfonctionnel, partiellement hydrophile, partiellement lipophile, ou un sel de

16

cet acide, possédant des sites réactifs ou réticulants espacés d'une distance qui équivaut environ à une chaîne carbonée normale contenant au moins 20 et pas plus de 360 atomes de carbone, dans une suspension ou solution aqueuse et un adjuvant facultatif,

caractérisé en ce que la substance formant le noyau est soit une matière pharmaceutique, soit une matière protéinique, comprenant un peptide biologiquement actif ou un polypeptide biologiquement actif, contenant de 14 à 100 aminoacides et le solvant organique, légèrement polaire est un solvant non dénaturant ou un complexe de solvants non dénaturant, suffisamment polaire pour orienter de manière anisotrope les composants saligènes et stabiliser le film de sel produit vis-à-vis d'une hydratation dans la phase aqueuse et capable de mettre la matière protéinique en solution ou en suspension sans en provoquer la désactivation.

2. Système suivant la revendication 1, caractérisé en ce que l'on choisit la base de Lewis dans le groupe formé par des amines monofonctionnelles, l'hexylamine, l'isopentylamine, la N-méthylpipéridine, la pipéridine, des amines difonctionnelles, la diméthyléthylènediamine, l'hexanediamine, la pipérazine, la triéthylènediamine, l'éthylènediamine, des amines polyfonctionnelles, le chlorure d'hexaméthylrosanilium, le chlorure de rosanilium, le chlorure de tétraméthylrosanilium, la mélamine, la tétraéthylènepentamine et la triéthylènetétramine et/ou on choisit l'acide de Lewis dans le groupe formé par la gélose, la gomme d'acacia, l'acide arabique, la carboxyméthylcellulose, la gomme ghatti, la gomme guar, la méthylcellulose, la cellulose oxydée, la pectine, la gomme adragante et le polyéthylèneglycol.

3. Système suivant la revendication 1 ou la revendication 2, caractérisé en ce que la matière protéinique est une toxine insecticide.

4. Système suivant la revendication 3, caractérisé en ce que la toxine insecticide provient de Bacillus thuringiensis.

5. Système suivant la revendication 1 ou la revendication 2, caractérisé en ce que la matière protéinique est l'insuline, le glucagon ou une hormone de croissance.

6. Système suivant l'une quelconque des revendications 1 à 5, caractérisé en ce que l'on choisit le solvant organique, légèrement polaire, dans le goupe formé par le bromoforme, le chloroforme, le dichlorométhane, le dichloréthane, l'éther diéthylique, l'éther diisopropylique, la méthyléthylcétone et le nitrobenzène.

7. Système suivant l'une quelconque des revendications 1 à 6, caractérisé en ce que le solvant non dénaturant est un ester cyclique ou un amide cyclique, ou linéaire, qui possède un paramètre de solubilité d'Hildebrand d'approximativement 23 à approximativement 32 MPa$^{1/2}$.

8. Système suivant la revendication 7, caractérisé en ce que l'ester cyclique est la butyrolactone, la valérolactone ou la caprolactone, l'amide cyclique est la N-méthyl-gamma-pyrolidone et l'amide linéaire est le N-méthylformamide, le N-méthylacétamide, leN,N-diméthylformamide, le N,N-diméthylacétamide ou le N-éthylacétamide.

9. Système suivant l'une quelconque des revendications 1 à 8, caractérisé en ce que le solvant ou le complexe de solvants, non dénaturant, comprend un premier solvant solubilisant et non dénaturant de la matière protéinique et un second solvant choisi dans le groupe formé par le chloroforme, le dichlorométhane, le dichloréthane et la méthyléthylcétone.

10. Système suivant l'une quelconque des revendications 1 à 9, caractérisé en ce que les groupements fonctionnels basiques et acides de l'acide de Lewis ou de la base de Lewis sont des réactifs sensiblement totalement substitués par un ion saligène et réagissent pour former le film de sel anisotrope précité et un sel neutre.

11. Système suivant l'une quelconque des revendications 1 à 10, caractérisé en ce que l'acide de Lewis possède un poids moléculaire d'au moins environ 12.000.

12. Système suivant l'une quelconque des revendications 1 à 11, caractérisé en ce que la paroi ou le noyau de la microcapsule comprend aussi un adjuvant promoteur de toxine, qui comprend un écran solaire, un agent de flottation ou ascentionnel, un agent de renforcement, ou un adhésif.

13. Système suivant la revendication 12, caractérisé en ce que le solvant non dénaturant comprend, de surcroît, un ester ou un amide qui favorise ou promouvoit l'encapsulation d'un écran solaire ou d'un agent de flottation ou ascentionnel comprenant un constituant de faible poids spécifique ou densité.

14. Système suivant l'une quelconque des revendications 1 à 13, caractérisé en ce que la substance qui constitue le noyau ou la paroi de la microcapsule comprend aussi un adjuvant comprenant un polymère de poids moléculaire élevé de l'acide acrylique, réticulé avec un polyalcénypolyéther, un polyoxyéthylène-polyoxypropylène ou un copolymère à blocs de ceux-ci, ou un adduct de poloxyéthylène de l'éthylènediamine.

15. Procédé de production d'un système débiteur ou distributeur suivant l'une quelconque des revendications 1 à 14, pour la libération graduelle d'une substance formant le noyau d'une microcapsule, comprenant un noyau et une paroi de microcapsule en un film de sel anisotrope,
caractérisé en ce que
on produit le système précité en faisant réagir, sous forme d'une émulsion,
    (a) un premier système réactionnel, qui comprend
        (i) un premier mélange réactif d'une base de Lewis ou d'un sel de cette base, qui possède une force basique (pKa) d'environ 7 à 12 et réagissant aux interfaces des phases organique et aqueuse d'une émulsion par liaison dipolaire et/ou ionique, dans un solvant de la base ou du sel, organique, légèrement polaire, le solvant organique, légèrement polaire, étant un solvant non dénaturant ou un complexe de solvants non dénaturant, qui est suffisamment polaire pour orienter de manière anisotrope les composants saligènes et stabiliser le film de sel produit vis-à-vis d'une hydratation dans la phase aqueuse et capable de mettre la substance formant le noyau en solution ou en suspension sans pour autant la désactiver, un adjuvant facultatif et une substance formant le noyau, ladite substance formant le noyau étant soit une matière pharmaceutique, soit une matière protéinique, qui comprend un peptide biologiquement actif ou un polypeptide biologiquement actif, qui contient de 14 à 100 aminoacides et qui peut être mis en solution ou en suspension dans le solvant organique précité, et
        (ii) un second mélange réactif d'un acide de Lewis polyfonctionnel, partiellement hydrophile, partiellement lipophile, ou d'un sel de celui-ci, qui possède des sites réactifs ou réticulants, espacés d'une distance qui équivaut à une chaîne carbonée normale contenant au moins 20 et pas plus de 360 atomes de carbone, dans une suspension ou une solution aqueuse et un adjuvant facultatif,
    ou
    (b) un second système réactif, qui comprend
        (i) un premier mélange réactif comprenant un sel d'une base de Lewis dans le solvant organique, légèrement polaire, de la base, un adjuvant facultatif et une substance constituant le noyau et
        (ii) un second mélange réactif comprenant un sel de l'acide de Lewis polyfonctionnel précité dans une suspension ou solution aqueuse et un adjuvant facultatif,
    ou
    (c) un troisième système réactif, qui comprend
        (i) un premier mélange réactif comprenant la base de lewis précitée ou un sel de celle-ci dans le solvant organique, légèrement polaire, de la base et la substance formant noyau précitée et
        (ii) un second mélange réactif comprenant l'acide de Lewis polyfonctionnel précité ou un sel de celui-ci dans une suspension ou solution aqueuse et
        (iii) un troisième mélange réactif comprenant un adjuvant formateur de paroi ou formateur de noyau, ou une combinaison d'un adjuvant formateur de paroi et d'un adjuvant formateur de noyau, de manière à former une substance qui constitue le noyau, microencapsulée dans un film de sel anisotrope à travers lequel la substance formant le noyau est graduellement perméable.

16. Procédé suivant la revendication 15, caractérisé en ce que l'on choisit la base de Lewis dans le groupe formé par des amines monofonctionnelles, l'hexylamine, l'isopentylamine, la N-méthylpipéridine, la pipéridine, des amines difonctionnelles, la diméthyléthylènediamine, l'hexanediamine, la pipérazine, la triéthylènediamine, l'éthylènediamine, des amines polyfonctionnelles, le chlorure d'hexaméthylrosanilium, le chlorure de rosanilium, le chlorure de tétraméthylrosanilium, la mélamine, la tétraéthylènepentamine et la triéthylènetétramine et/ou on choisit l'acide de Lewis dans le groupe formé par la gélose, la

EP 0 299 205 B1

gomme d'acacia, l'acide arabique, la carboxyméthylcellulose, la gomme ghatti, la gomme guar, la méthylcellulose, la cellulose oxydée, la pectine, la gomme adragante et le polyéthylèneglycol.

**17.** Procédé suivant la revendication 15 et la revendication 16, caractérisé en ce que l'on choisit le solvant organique, légèrement polaire, dans le goupe formé par le bromoforme, le chloroforme, le dichlorométhane, le dichloréthane, l'éther diéthylique, l'éther diisopropylique, la méthyléthylcétone et le nitrobenzène.

**18.** Procédé suivant l'une quelconque des revendications 15 à 17, caractérisé en ce que la matière protéinique est une toxine insecticide.

**19.** Procédé suivant la revendication 18, caractérisé en ce que la toxine insecticide dérive de Bacillus thuringiensis.

**20.** Procédé suivant l'une quelconque des revendications 15 à 19, caractérisé en ce que la base de Lewis ou son sel est polyfonctionnel.

**21.** Procédé suivant l'une quelconque des revendications 15 à 20, caractérisé en ce que l'on produit le système précité en faisant réagir le premier et le second mélanges réactifs de la revendication 15 (b).

**22.** Procédé suivant la revendication 21, caractérisé en ce que le sel de la base de Lewis précitée est le chlorure de benzalconium ou le chlorure de cétylpyridinium et en ce que le sel de l'acide de Lewis polyfonctionnel précité est la carboxyméthylcellulose sodique, un sel de sodium d'un acide polyacrylique ou un sel de sodium d'un acide polyacrylique à réticulation polyoxyéthylénique.

**23.** Procédé suivant l'une quelconque des revendications 15 à 20, caractérisé en ce que l'on produit le système précité en faisant réagir le premier, le second et le troisième mélange réactifs de la revendication 15 (c).

**24.** Procédé suivant la revendication 23, caractérisé en ce que le troisième réactif de la revendication 15 (c) (iii) est un adjuvant formateur de paroi.

**25.** Procédé suivant la revendication 23, caractérisé en ce que le troisième réactif de la revendication 15 (c) (iii) est un adjuvant formateur de noyau.

**26.** Procédé suivant l'une quelconque des revendications 23 à 25, caractérisé en ce que l'adjuvant formateur de paroi ou l'adjuvant formateur de noyau est un polymère d'acide acrylique, de poids moléculaire élevé, réticulé avec un polyalcénypolyéther, un polyoxyéthylène-polyoxypropylène, ou un copolymère à blocs de ceux-ci, ou un adduct polyoxyéthylénique de l'éthylènediamine.

**Patentansprüche**

**1.** Abgabe-System für die allmähliche Freisetzung einer Kernsubstanz aus einer Mikrokapsel, die einen Kern und eine Mikrokapselwand aus einem anisotropen Salzfilm umfaßt, der das Reaktionsprodukt ist aus

(a) einer Lewis-Base oder einem Salz davon, das eine Basenstärke ($pK_a$) von etwa 7 bis 12 hat und an den Grenzflächen der organischen und wäßrigen Phase einer Emulsion durch Dipol- und/oder ionische Bindung reagiert, in einem schwach polaren organischen Lösungsmittel für die Base oder das Salz und einem möglichen Hilfsstoff und

(b) einer polyfunktionellen, partiell hydrophilen, partiell lipophilen Lewis-Säure oder einem Salz davon, das reaktive oder vernetzende Stellen im Molekül aufweist, die in einer Entfernung räumlich angeordnet sind, die einer normalen Kohlenstoffkette equivalent ist, die wenigstens 20 und nicht mehr als 360 Kohlenstoffatome enthält, in einer wäßrigen Lösung oder Suspension und einem möglichen Hilfsstoff,

dadurch gekennzeichnet, daß die Kernsubstanz entweder ein pharmazeutisches Material oder ein proteinartiges Material ist, welches ein biologisch aktives Peptid oder Polypeptid umfaßt, das 14 bis 100 Aminosäuren enthält, und daß das schwach polare organische Lösungsmittel ein nicht-denaturierendes Lösungsmittel oder Lösungsmittelkomplex ist, das/der ausreichend polar ist, um die salzbildenden Komponenten anisotrop zu orientieren und den als Produkt entstehenden Salzfilm gegen Hydratation in der wäßrigen Phase zu stabilisieren und das proteinartige Material, ohne es zu desaktivieren, solubilisieren oder suspendieren kann.

2. Abgabesystem nach Anspruch 1, worin die Lewis-Base gewählt ist aus der aus monofunktionellen Aminen, Hexylamin, Isopentylamin, N-Methylpiperidin, Piperidin, difunktionellen Aminen, Dimethylethylendiamin, Hexandiamin, Piperazin, Triethylendiamin, Ethylendiamin, polyfunktionellen Aminen, Hexamethylrosaniliumchlorid, Rosaniliumchlorid, Tetramethylrosaniliumchlorid, Melamin, Tetraethylenpentamin und Triethylentetramin bestehenden Gruppe, und/oder daß die Lewis-Säure gewählt ist aus der aus Agar, Gummi arabicum, Arabinsäure, Carboxymethylcellulose, Ghattigummi, Guargummi, Methylcellulose, oxidierte Cellulose, Pektin, Traganth und Polyethylenglykol bestehenden Gruppe.

3. Abgabesystem nach Anspruch 1 oder Anspruch 2, worin das proteinartige Material ein Insektizid-Toxin ist.

4. Abgabesystem nach Anspruch 3, worin das Insektizid-Toxin von Bacillus thuringiensis abgeleitet ist.

5. Abgabesystem nach Anspruch 1 oder Anspruch 2, worin das proteinartige Material Insulin, Glucagon oder ein Wachstumshormon ist.

6. Abgabesystem nach irgendeinem der Ansprüche 1 bis 5, worin das schwach polare organische Lösungsmittel gewählt ist aus der aus Bromoform, Chlorofrom, Dichlormethan, Dichlorethan, Diethylether, Diisopropylether, Methylethylketon und Nitrobenzol bestehenden Gruppe.

7. Abgabesystem nach Irgendeinem der Ansprüche 1 bis 6, worin das nicht-denaturierende Lösungsmittel ein zyklischer Ester oder ein zyklisches oder lineares Amid ist, das einen Hildebrand-Löslichkeitsparameter von angenähert 23 bis angenähert 32 MPa$^{1/2}$ aufweist.

8. Abgabesystem nach Anspruch 7, worin der zyklische Ester Butyrolacton, Valerolacton oder Caprolacton, das zyklische Amid N-Methyl-gamma-pyrrolidon und das lineare Amid N-Methylformamid, N-Methylacetamid, N,N-Dimethylformamid, N,N-Dimethylacetamid oder N-Ethylacetamid ist.

9. Abgabesystem nach irgendeinem der Ansprüche 1 bis 8, worin das nicht-denaturierende Lösungsmittel oder der Lösungsmittelkomplex ein erstes, nicht-denaturierendes, solubilisierendes Lösungsmittel für das proteinartige Material und ein zweites Lösungsmittel umfaßt, das gewählt ist aus der aus Chloroform, Dichlormethan, Dichlorethan und Methylethylketon bestehenden Gruppe.

10. Abgabesystem nach irgendeinem der Ansprüche 1 bis 9, worin die basischen und sauren funktionellen Einheiten der Lewis-Säure oder der Lewis-Base Reaktanden sind, die im wesentlichen vollständig mit einem salzbildenden Ion substituiert sind und unter Bildung des anisotropen Salzfilms und eines neutralen Salzes reagieren.

11. Abgabesystem nach irgendeinem der Ansprüche 1 bis 10, worin die Lewis-Säure ein Molekulargewicht von wenigstens etwa 12.000 aufweist.

12. Abgabesystem nach irgendeinem der Ansprüche 1 bis 11, worin die Mikrokapselwand oder der Kern außerdem ein toxinförderndes Hilfsmittel einschließt, das einen Sonnenschutz, ein Auftriebsmittel, ein Verstärkungsmittel oder ein Klebemittel umfaßt.

13. Abgabesystem nach Anspruch 12, worin das nicht-denaturierende Lösungsmittel außerdem einen Ester oder ein Amid einschließt, welches die Verkapselung eines Sonnenschutzes oder eines Auftriebsmittels fördert, welches einen Bestandteil mit niedriger Dichte umfaßt.

14. Abgabesystem nach irgendeinem der Ansprüche 1 bis 13, worin die Substanz für die Mikrokapselwand oder den Kern außerdem ein Hilfsmittel einschließt, das ein Polymer von Acrylsäure mit hohem Molekulargewicht umfaßt, das mit einem Polyalkenylpolyether, einem Polyoxyethylenpolyoxypropylen oder einem Blockcopolymer daraus oder einem Polyoxyethylen-Addukt von Ethylendiamin vernetzt ist.

20

**15.** Verfahren zur Herstellung eines Abgabesystems nach irgendeinem der Ansprüche 1 bis 14 für die allmähliche Freisetzung einer Kernsubstanz aus einer Mikrokapsel, welche einen Kern und eine Mikrokapselwand aus einem anisotropen Salzfilm umfaßt, dadurch gekennzeichnet, daß das Abgabesystem dadurch hergestellt wird, daß man in Form einer Emulsion miteinander umsetzt

(a) ein erstes Reaktionssystem, welches umfaßt

(i) eine erste Reaktandenmischung aus einer Lewis-Base oder einem Salz daraus, welche(s) eine Basenstärke ($pK_a$) von etwa 7 bis 12 aufweist und an den Grenzflächen der organischen und der wäßrigen Phase einer Emulsion über Dipol- und/oder ionische Bindung reagiert, in einem schwach polaren organischen Lösungsmittel für die Base oder das Salz, wobei das schwach polare organische Lösungsmittel ein nicht-denaturierendes Lösungsmittel oder Lösungsmittelkomplex ist, das/der ausreichend polar ist, um die salzbildenden Komponenten anisotrop zu orientieren und den als Produkt gebildeten Salzfilm gegen Hydratation in der wäßrigen Phase zu stabilisieren und die Kernsubstanz ohne deren Desaktivierung solubilisieren oder suspendieren kann, einem möglichen Hilfsmittel und einer Kernsubstanz, wobei die Kernsubstanz entweder ein pharmazeutisches Material oder ein proteinartiges Material ist, das ein biologisch aktives Peptid oder Polypeptid umfaßt, welches 14 bis 100 Aminosäuren enthält und in dem organischen Lösungsmittel solubilisierbar oder suspendierbar ist, mit

(ii) einer zweiten Reaktandenmischung aus einer polyfunktionellen, partiell hydrophilen, partiell lipophilen Lewis-Säure oder einem Salz davon, welche(s) reaktive oder vernetzende Molekülstellen aufweist, die in einer Entfernung räumlich angeordnet sind, die einer normalen Kohlenstoffkette equivalent ist, die wenigstens 20 und nicht mehr als 360 Kohlenstoffatome enthält, in einer wäßrigen Lösung oder Suspension und einem möglichen Hilfsstoff, oder

(b) ein zweites Reaktionssystem, welches umfaßt

(i) eine erste Reaktandenmischung, welche ein Salz der Lewis-Base in dem schwach organischen Lösungsmittel für die Base, einen möglichen Hilfsstoff und die Kernsubstanz umfaßt, mit

(ii) einer zweiten Reaktandenmischung, welche ein Salz der polyfunktionellen Lewis-Säure in einer wäßrigen Lösung oder Suspension und einen möglichen Hilfsstoff umfaßt, oder

(c) ein drittes Reaktionssystem, das umfaßt

(i) eine erste Reaktandenmischung, die die Lewis-Base oder ein Salz davon in dem schwach polaren organischen Lösungsmittel für die Base und die Kernsubstanz umfaßt, mit

(ii) einer zweiten Reaktandenmischung, welches die polyfunktionelle Lewis-Säure oder ein Salz davon in einer wäßrigen Lösung oder Suspension umfaßt, und mit

(iii) einer dritten Reaktandenmischung, die einen die Wand bildenden oder den Kern bildenden Hilfsstoff oder eine Kombination davon umfaßt,

unter Bildung einer Kernsubstanz, die in einem anisotropen Salzfilm mikroverkapselt ist, durch den die Kernsubstanz allmählich durchtreten kann.

**16.** Verfahren nach Anspruch 15, worin die Lewis-Base gewählt ist aus der aus monofunktionellen Aminen, Hexylamin, Isopentylamin, N-Methylpiperidin, Piperidin, difunktionellen Aminen, Dimethylethylendiamin, Hexandiamin, Piperazin, Triethylendiamin, Ethylendiamin, polyfunktionellen Aminen, Hexamethylrosaniliumchlorid, Rosaniliumchlorid, Tetramethylrosaniliumchlorid, Melamin, Tetraethylenpentamin und Triethylentetramin bestehenden Gruppe, und/oder daß die Lewis-Säure gewählt ist aus der aus Agar, Gummi arabicum, Arabinsäure, Carboxymethylcellulose, Ghattigummi, Guargummi, Methylcellulose, oxidierte Cellulose, Pektin, Traganth und Polyethylenglykol bestehenden Gruppe.

**17.** Verfahren nach Anspruch 15 und Anspruch 16, worin das schwach polare organische Lösungsmittel gewählt ist aus der aus Bromoform, Chlorofrom, Dichlormethan, Dichlorethan, Diethylether, Diisopropylether, Methylethylketon und Nitrobenzol bestehenden Gruppe.

**18.** Verfahren nach irgendeinem der Ansprüche 15 bis 17, worin das proteinartige Material ein Insektizid-Toxin ist.

**19.** Verfahren nach Anspruch 18, worin das Insektizid-Toxin von Bacillus thuringiensis abgeleitet ist.

**20.** Verfahren nach irgendeinem der Ansprüche 15 bis 19, worin die Lewis-Base oder deren Salz polyfunktionell ist.

**21.** Verfahren nach irgendeinem der Ansprüche 15 bis 20, worin das Abgabesystem dadurch hergestellt

21

EP 0 299 205 B1

wird, daß man die erste und zweite Reaktandenmischung aus Anspruch 15 (b) miteinander umsetzt.

22. Verfahren nach Anspruch 21, worin das Salz der Lewis-Base Benzalkoniumchlorid oder Cetylpyridiniumchlorid ist, und worin das Salz der polyfunktionellen Lewis-Säure Natriumcarboxymethylcellulose, ein Natriumsalz von Polyacrylsäure oder ein Natriumsalz von Polyoxyethylen-vernetzter Polyacrylsäure ist.

23. Verfahren nach irgendeinem der Ansprüche 15 bis 20, worin das Abgabesystem dadurch hergestellt wird, daß man die erste, zweite und dritte Reaktandenmischung aus Anspruch 15 (c) miteinander umsetzt.

24. Verfahren nach Anspruch 23, worin der dritte Reaktand aus Anspruch 15 (c) (iii) ein die Wand bildender Hilfsstoff ist.

25. Verfahren nach Anspruch 23, worin der dritte Reaktand aus Anspruch 15 (c) (iii) ein den Kern bildender Hilfsstoff ist.

26. Verfahren nach irgendeinem der Ansprüche 23 bis 25, worin der die Wand bildende oder der den Kern bildende Hilfsstoff ein Polymer hohen Molekulargewichts von Acrylsäure ist, das mit einem Polyalkenylpolyether, einem Polyoxyethylenpolyoxypropylen oder einem Blockcopolymer daraus oder einem Polyoxyethylen-Addukt von Ethylendiamin vernetzt ist.

22